# EUROPÄISCHE PATENTANMELDUNG

(11) **EP 1 566 154 A1**
(43) Veröffentlichungstag der Anmeldung: **24.08.2005**
(21) Anmeldenummer: 04003943.0
(22) Anmeldetag: 20.02.2004
(51) Int. Cl.: A61F 2/36, A61F 2/40

(54) **Prothese zum Oberflächenersatz im Bereich der Kugel von Kugelgelenken**

(71) Anmelder: Argomedical AG, 6330 Cham (CH)
(72) Erfinder: Kropf, Philipp, 6330 Cham (CH); Hertel, Ralph, 3074 Muri (CH)
(74) Vertreter: Blum, Rudolf Emil

(57) **Zusammenfassung**

Die Prothese (1) zum Oberflächenersatz im Bereich der Kugel von Kugelgelenken weist einen Kugelschalenausschnitt (2) auf, dessen Aussenfläche (3) für das Aufliegen in einer Gelenkpfanne ausgestaltet ist. Der Kugelschalenausschnitt (2) bildet ferner einen Hohlraum mit einer Öffnung, so dass er auf ein durch partielle Resektion vorbereitetes Knochenende aufsetzbar ist. Zur Befestigung am Knochen ist eine zumindest teilweise im Hohlraum angeordnete Krone (5) vorgesehen. Die Prothese (1) hat eine grosse Auflagefläche und greift insbesondere in die tragenden Strukturen des Knochens. Ihre Implantation erfordert nur eine geringe Knochenresektion und ist minimal invasiv.

## Beschreibung

Die Erfindung betrifft eine Prothese gemäss Oberbegriff des Anspruch 1, ein Verfahren zur Implantation einer solchen Prothese gemäss Oberbegriff von Anspruch 15 und ferner einen Materialsatz zur Herstellung einer besonderen Ausführung einer solchen Prothese gemäss Anspruch 16.

Prothesen dieser Art werden bei Kugelgelenken, insbesondere bei Schulter- und Hüftgelenk, eingesetzt. Grundsätzlich gibt es bei Prothesen für Kugelgelenke zwei unterschiedliche Lösungsvarianten.

Bei einer ersten Variante wird die Kugel ganz oder zumindest zur Hälfte abgetrennt und durch eine Prothese ersetzt, wobei diese einen Schaft aufweist, der in den verbleibenden Knochen bzw. dessen Markraum eingeführt wird. Derartige Schaftprothesen eignen sich besonders für ältere Menschen und/oder im Fall von Knochenbrüchen, wie z.B. Oberschenkelhalsbrüchen.

Bei einer zweiten Variante wird lediglich die Oberfläche des Knochens bzw. Knorpels zumindest teilweise ersetzt. Man spricht in diesem Fall von "Oberflächenersatzprothesen", "Resurfacing-Prothesen" oder auch "Kappenprothesen". Entsprechende Prothesen werden z.B. unter den Warenzeichen "Durom" und "Copeland" angeboten. Diese Prothesenart hat den Vorteil, dass ihre Implantation minimal invasiv ist. Die Operationsdauer ist kürzer. Bei einem Ersatz der Prothese gibt es mehr Möglichkeiten. Insbesondere bietet sich eine Rückzugsoperation an, bei der die Prothese durch eine Schaftprothese, wie sie oben beschrieben ist, ersetzt wird.

Ein Problem bei Oberflächenersatzprothesen besteht jedoch darin, dass ihre Befestigung meist schwieriger ist als bei Prothesen mit Schaft. Es gibt verschiedene Ansätze die Befestigung zu realisieren.

So gibt es Oberflächenersatzprothesen, welche nur aus einer Kappe bestehen, dafür aber im Wesentlichen die gesamte Gelenkkugel umschliessen. Dies führt zumindest dem Anschein nach zu einer besseren Verteilung der Kräfte, es muss jedoch mehr Knochenmaterial abgetragen werden und die Stabilität bei Zugkräften ist unter Umständen unvollkommen.

Ferner gibt es Oberflächenprothesen, die einen Stiel aufweisen, wobei dessen Länge in etwa dem Kugeldurchmesser entspricht. Dadurch werden die Kräfte auf zusätzliche Kontaktflächen verteilt und die Prothese ist auf der Gelenkkugel besser zentriert. Die Lösung ist jedoch unvollkommen, unter anderem weil der Stiel zu Hebelkräften und damit unter Umständen zu einer Lockerung der Prothese führen kann.

In einigen Fällen stellt sich bei den bekannten Prothesen auch das Problem, dass der Knochen im Bereich des Gelenkkopfes und damit insbesondere bei den Kontaktflächen mit der Prothese abstirbt.

Es stellt sich daher die Aufgabe, eine Prothese der eingangs genannten Art bereitzustellen, die die oben genannten Nachteile zumindest teilweise vermeidet.

Diese Aufgabe wird von Anspruch 1 gelöst, indem die Prothese eine Krone aufweist, welche einen von einem Kugelschalenausschnitt zur Aufnahme eines Knochenendes gebildeten Hohlraum im Wesentlichen in einen ersten und einen zweiten Hohlraum unterteilt.

Eine derartige Prothese hat den Vorteil, dass ihre Befestigung stabil ist und aufgrund der grossen Auflagefläche die zwischen Knochen und Prothese wirkenden Kräfte gut verteilt sind. Ferner greift die Krone bei geeigneter Anordnung und Dimensionierung direkt in die tragenden und intakten Strukturen des Knochens womit eine langfristig gute Stabilität erreicht wird. Die Implantation der Prothese ist wenig invasiv. Es muss nur wenig Knochenmaterial entfernt werden und es bleibt damit viel Knochen erhalten. Die Operationsdauer ist relativ kurz.

Weitere Vorteile und bevorzugte Ausführungsbeispiele ergeben sich aus den abhängigen Ansprüchen, sowie aus der nun folgenden Beschreibung anhand der Figuren. Dabei zeigen:
Fig. 1 einen Schnitt durch eine bevorzugte Ausführung der erfindungsgemässen Prothese,
Fig. 2 eine perspektivische Ansicht der bevorzugten Ausführung der erfindungsgemässen Prothese von Fig. 1.

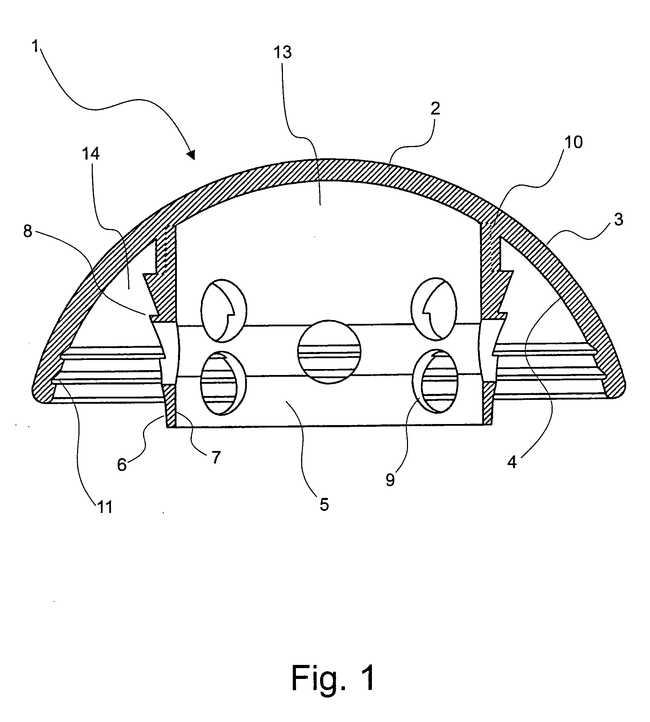

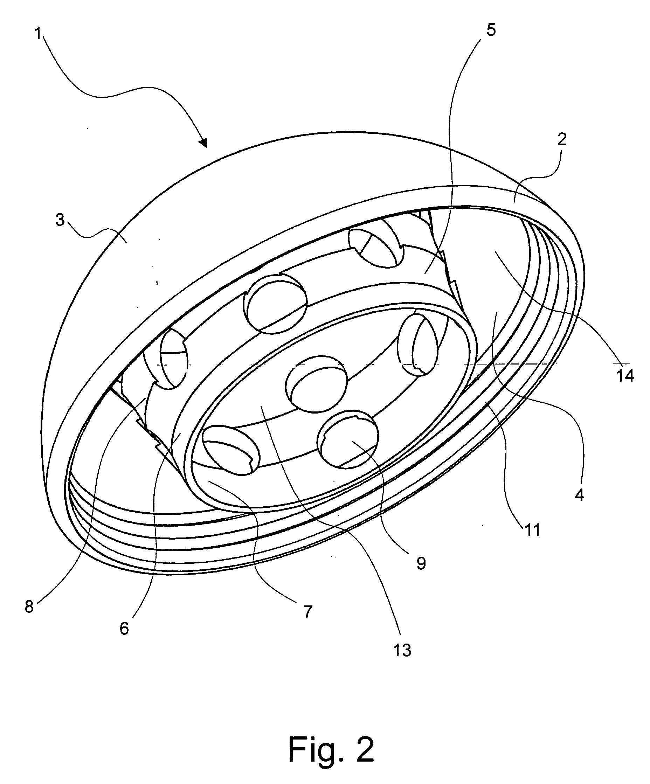

Fig. 1 zeigt einen Schnitt durch eine bevorzugte Ausführung der erfindungsgemässen Prothese. Fig. 2 zeigt eine perspektivische Ansicht derselben Prothese. Die Prothese dient zum teilweisen Ersatz der Oberfläche einer Kugel eines Kugelgelenkes, wie z.B. dem Oberschenkelkopf. Die Prothese 1 besteht im Wesentlichen aus einem Kugelschalenausschnitt 2 und einer darin angeordneten Krone 5.

Der Kugelschalenausschnitt 2 hat bevorzugt im Wesentlichen die Form einer Halbkugel oder die Form eines Ausschnittes einer Halbkugel. Dies hat den Vorteil, dass bei der Implantation nur eine partielle Resektion der Gelenkkugeloberfläche vorgenommen werden muss. Der Kugelschalenausschnitt 2 ist bevorzugt etwas kleiner als eine Halbkugel, d.h. er erstreckt sich vorzugsweise über einen Winkel von 145 bis 180 Grad. Der Kugelschalenausschnitt 2 kann auch als "Kappe" oder, wenn er wie in der gezeigten Ausführung im Wesentlichen halbkugelförmig ist, als "Hemisphäre" bezeichnet werden. Die Aussenfläche 3 des Kugelschalenausschnittes 2 ist zum Aufliegen in einer Gelenkpfanne ausgestaltet. Die Aussenfläche 3 ist insbesondere glatt, so dass sie sich in der Gelenkpfanne gut bewegen kann. Bei der Gelenkpfanne kann es sich ebenfalls um eine Prothese handeln. Auf der Innenfläche 4 ist eine Riffelung 11 vorgesehen, welche durch im Bereich des Randes verlaufende Ringnuten gebildet ist. Eine Ausgestaltung des Kugelschalenausschnittes 2 mit einem derartigen oder anders gearteten Relief führt zu einem besseren Halt der Prothese.

Die Krone 5 dient zur Befestigung bzw. Verankerung der Prothese am bzw. im Knochen und/oder zur besseren Verteilung der Auflagekräfte. Sie hat im Wesentlichen die Form einer Zylindermantelfläche. Ihr Durchmesser beträgt 45 bis 75 Prozent, insbesondere etwa 60 Prozent, des Durchmessers des Kugelschalenausschnittes 2. Durch diese Dimensionierung greift die Krone 5 in bzw. an einen sehr stabilen Teil des Knochens, wobei dessen röhrenartige Struktur berücksichtigt wird. Die Krone 5 weist eine Aussenfläche 6 und eine Innenfläche 7 auf. Auf der Aussenfläche 6 ist eine Riffelung 8 in Form von um die Krone 5 herumführenden Ringwülsten vorgesehen. Die Riffelung kann auch auf der Innenfläche 7 oder auf beiden Flächen 6, 7 vorgesehen sein. Eine Ausgestaltung der Krone 5 mit einem derartigen oder anders gearteten Relief führt zu einem besseren Halt der Prothese. Die Krone 5 ist zumindest teilweise im Kugelschalenausschnitt 2 angeordnete. Durch die Krone 5 ist der vom der Kugelschalenausschnitt 2 zur Aufnahme eines Knochenendes gebildete offene Hohlraum im Wesentlichen in einen inneren, ersten Hohlraum 13 und einen äusseren, ringförmig darum herum angeordneten, zweiten Hohlraum 14 unterteilt. Der Ausdruck "im Wesentlichen" soll in diesem Zusammenhang anzeigen, dass die Krone 5, wie weiter unten näher erläutert, Aussparungen aufweisen kann, welche die beiden Hohlräume 13 und 14 verbinden. Die Hohlräume 13, 14 dienen der Aufnahme des Knochenendes und sind daher auf der dem Knochen zugewandten Seite offen. Die Öffnung des ersten Hohlraums 13 ist kreisflächenförmig, die des zweiten Hohlraumes 14 ist ringförmig. Im Falle der implantierten Prothese steht der Knochen in diese Hohlräume 13, 14 hinein. Insbesondere nach einer Verwachsungsphase sind die Hohlräume im Wesentlichen ganz mit Knochen oder knochenähnlichem Gewebe ausgefüllt. In der Krone 5 sind bevorzugt ca. fünf bis zehn Aussparungen 9, insbesondere im Wesentlichen runde Löcher, vorgesehen. Durch diese kann der in den beiden Hohlräumen 13, 14 befindliche Knochen hindurch und zusammenwachsen. Dies ermöglicht eine bessere Durchblutung des Knochens und führt zu einem besseren Halt der Prothese 1, insbesondere auch bei Zugkräften. Die Aussparungen können sich auch über die ganze Länge der Krone erstrecken, so dass die Krone durch Spalte unterbrochen ist und/oder in einzelne Zacken unterteilt ist. Die Innenfläche 4 des Kugelschalenausschnittes 2 und die Oberfläche, d.h. die Aussenfläche 6 und Innenfläche 7, der Krone 5 sind für einen Kontakt und/oder ein Verwachsen mit dem Knochen ausgestaltet. Die Flächen sind hierfür insbesondere rauh.

Die Krone 5 ist bevorzugt mit dem Kugelschalenausschnitt 2 zusammen einstückig ausgestaltet. Krone 5 und Kugelschalenausschnitt 2 können jedoch auch zunächst getrennt gefertigt und anschliessend an einer Verbindungsstelle 10 verschweisst sein. Die Krone 5 ist koaxial zum kreisförmigen Rand des Kugelschalenausschnittes 2 angeordnet, d.h. die Symmetrieachsen von Krone 5 und Kugelschalenausschnitt 2 liegen auf derselben Gerade. Der Rand der Krone 5 verläuft bevorzugt im Wesentlichen in derselben Ebene wie der Rand des Kugelschalenausschnittes 2. Bei der gezeigten Ausführung steht der Rand der Krone um 1 bis 3 mm über die Ebene hinaus, in welcher der Rand des Kugelschalenausschnittes 2 verläuft. So ist sichergestellt, dass beim Aufsetzen der Prothese die Krone 5 den vorbereiteten Knochen zuerst kontaktiert und eine für die Krone 5 vorbereitete Nut als Ausrichtungs- bzw. Zentrierhilfe verwendet werden kann. Die Ausgestaltung mit einer nicht wesentlich über den Rand des Kugelschalenausschnittes 2 hinausstehenden Krone 5 hat den Vorteil, dass, falls ein Ersatz der Prothese durch eine Schaftprothese notwendig ist, die bestehende Prothese nicht aus dem Knochen herausgelöst werden muss, sondern einfach zusammen mit einem Teil des Knochens abgesägt werden kann. Es muss dabei nur Knochen durchsägt werden. Der knochenseitige Teil der Gelenkkugel bleibt in einem für eine Schaftprothese ausreichendem Masse stehen.

Die erfindungsgemässe Prothese hat den Vorteil, dass sie konsequent die erforderliche Knochenresektion minimiert. Dies vergrössert insbesondere die Auswahl möglicher Rückzugsoperationen.

Bei der Implantation der erfindungsgemässen Prothese wird zunächst der Kopf des Knochens mit einem Spezialwerkzeug entsprechend der Form des Kugelschalenausschnittes abgeschält und/oder abgefräst. Anschliessend wird eine Ringnut in den Knochen geschnitten, welche auf die Abmessungen der Krone abgestimmt ist. Dies erfolgt bevorzugt mit einem eigens dafür vorgesehenen Kronenfräser. Dann wird die Prothese auf den derart vorbereiteten Knochen aufgedrückt und/oder in diesen eingeschlagen. Es ist kein Zement erforderlich. Eine typische Operationszeit ist bei Verwendung der erfindungsgemässen Prothese ca. 20 min, wohingegen die Operationszeit bei Verwendung einer Schaftprothese in der Grössenordnung von einer Stunde liegt.

Die erfindungsgemässe Prothese kann für Kugelgelenke allgemein verwendet werden, insbesondere jedoch für das Schulter und das Hüftgelenk. Die Prothese wird hierfür in verschiedenen Grössen bereitgestellt. Die Prothese wird ferner in verschiedenen Grössen bereitgestellt, um eine auf die Anatomie bzw. die Körpermasse des Patienten optimal abgestimmte Prothese aussuchen zu können.

In einer weiteren Ausführung der Erfindung werden Kugelschalenausschnitt und Krone separat, d.h. als Einzelteile bzw. als Materialsatz von Einzelteilen bereitgestellt. Am Kugelschalenausschnitt und/oder an der Krone ist dabei ein mechanisches Befestigungsmittel wie z.B. ein Schraubgewinde, ein Bajonettverschluss oder eine Klemmvorrichtung vorgesehen. Mittels dieser lassen sich Kugelschalenausschnitt und Krone verbinden. Bei einer solchen Ausführung als Materialsatz ist es möglich verschieden ausgestaltete Kugelschalen, insbesondere mit verschiedenen Kugelradien, und verschieden ausgestaltete Kronen, insbesondere mit verschiedenen Längen und Durchmessern, in Abstimmung auf die Anatomie des Patienten miteinander zu kombinieren. Es können so, insbesondere während der Operation, speziell angepasste Prothesen hergestellt werden.

In einer weiteren Ausführung der Erfindung ist die Prothese statt zum Einschlagen bzw. Aufdrücken zum Ein- bzw. Aufschrauben ausgestaltet. Die Riffelung auf der Krone und/oder die Riffelung auf der Innenfläche des Kugelschalenausschnittes ist hierfür als Gewinde ausgebildet. Ferner weist die Prothese insbesondere Kerben und/oder Abflachungen als Angriffsfläche für ein Drehwerkzeug auf.

Bei der in den Figuren gezeigten Ausführung hat die Krone im Wesentlichen die Form einer Zylindermantelfläche. Die Krone, bzw. ihre Innen- und Aussenfläche, kann jedoch auch ganz oder teilweise konisch ausgestaltet werden. In einer möglichen Variante ist die Innenfläche im Wesentlichen zylindrisch und die Aussenfläche im Wesentlichen konisch. In einer weiteren Variante ist die Innenfläche im Wesentlichen konisch und die Aussenfläche im Wesentlichen zylindrisch. In einer weiteren Variante sind sowohl die Innenfläche als auch die Aussenfläche im wesentlichen konisch. Die Konizität ist dabei bevorzugt so, dass die Wandstärke der Krone zum Kugelschalenausschnitt hin zunimmt, z.B. von 1 mm am Rand bis auf 4 mm am Übergang zum Kugelschalenausschnitt. Dies hat den Vorteil, dass beim Aufsetzen der Prothese zusätzlicher Druck erzeugt wird, was den Halt der Prothese auf dem Knochen verbessert.

Ferner hat bei der in den Figuren gezeigten Ausführung die Krone den Grundriss eines Kreises. Der Grundriss kann jedoch auch anders gewählt werden, z.B. sternförmig oder in Form eines insbesondere gleichmässigen Vielecks, z.B. eines Sechsecks.

Während anhand der Figuren im Wesentlichen nur eine bevorzugte Ausführung der erfindungsgemässen Prothese beschrieben wurde ist es dem Fachmann klar, dass die meisten der aufgeführten Merkmale unter Berücksichtigung ihrer Funktion variiert, anders kombiniert oder weggelassen werden können.

## Patentansprüche

1. Prothese zum Oberflächenersatz im Bereich einer Kugel von Kugelgelenken, wobei die Prothese (1) einen Kugelschalenausschnitt (2) mit einer zum Aufliegen in einer Gelenkpfanne ausgestalteten Aussenfläche (3) aufweist, **dadurch gekennzeichnet, dass** zur Befestigung und/oder zur besseren Verteilung von Auflagekräften eine Krone (5) vorgesehen ist, welche einen vom Kugelschalenausschnitt (2) zur Aufnahme eines Knochenendes gebildeten Hohlraum im Wesentlichen in einen ersten Hohlraum (13) und einen zweiten Hohlraum (14) unterteilt.

2. Prothese gemäss Anspruch 1, **dadurch gekennzeichnet, dass** der Kugelschalenausschnitt (2) im Wesentlichen eine Form einer Halbkugel oder eines Ausschnittes einer Halbkugel hat.

3. Prothese gemäss einem der vorhergehenden Ansprüche, **dadurch gekennzeichnet, dass** eine Innenfläche (4) des Kugelschalenausschnittes (2) und/oder eine Oberfläche der Krone (5) für einen Kontakt und/oder ein Verwachsen mit einem Knochen ausgestaltet ist und hierfür insbesondere rauh ist.

4. Prothese gemäss einem der vorhergehenden Ansprüche, **dadurch gekennzeichnet, dass** die Krone (5) mindestens eine Aussparung (9) aufweist, insbesondere fünf bis zehn Aussparungen (9).

5. Prothese gemäss einem der vorhergehenden Ansprüche, **dadurch gekennzeichnet, dass** die Krone (5) auf einer Aussenfläche (6) und/oder Innenfläche (7) ein Relief aufweist, insbesondere eine Riffelung (8), welche aus um die Krone (5) herumführende Ringwülsten gebildet ist.

6. Prothese gemäss einem der vorhergehenden Ansprüche, **dadurch gekennzeichnet, dass** der Kugelschalenausschnitt (2) auf einer Innenfläche (4) ein Relief aufweist, insbesondere eine Riffelung, welche entlang eines Randes des Kugelschalenausschnitts (2) verläuft.

7. Prothese gemäss einem der vorhergehenden Ansprüche, **dadurch gekennzeichnet, dass** sie zum Auf- bzw. Einschrauben ausgestaltet ist, wobei die Krone (5) und/oder eine Innenfläche (4) des Kugelschalenausschnittes (2) ein Gewinde aufweist und die Prothese (1) insbesondere Kerben und/oder Abflachungen als Angriffsfläche für ein Drehwerkzeug aufweist.

8. Prothese gemäss einem der vorhergehenden Ansprüche, **dadurch gekennzeichnet, dass** ein Grundriss der Krone (5) eine Form eines Kreises oder eines im wesentlichen gleichmässigen Vieleckes hat.

9. Prothese gemäss Anspruch 8, **dadurch gekennzeichnet, dass** die Krone (5) im Wesentlichen koaxial zu einem Rand von Kugelschalenausschnitt (2) angeordnet ist.

10. Prothese gemäss einem der vorhergehenden Ansprüche, **dadurch gekennzeichnet, dass** ein Durchmesser der Krone (5) 45 bis 75 Prozent, insbesondere etwa 60 Prozent, eines Durchmessers des Kugelschalenausschnittes (2) beträgt.

11. Prothese gemäss einem der vorhergehenden Ansprüche, **dadurch gekennzeichnet, dass** ein Rand der Krone (5) im Wesentlichen in derselben Ebene verläuft wie ein Rand des Kugelschalenausschnittes (2).

12. Prothese gemäss einem der vorhergehenden Ansprüche, **dadurch gekennzeichnet, dass** ein Rand der Krone (5) um 1 bis 3 mm über eine Ebene hinaussteht, in welcher ein Rand des Kugelschalenausschnittes (2) verläuft.

13. Prothese gemäss einem der vorhergehenden Ansprüche, **dadurch gekennzeichnet, dass** Kugelschalenausschnitt (2) und Krone (5) zusammen einstückig ausgebildet sind und/oder miteinander verschweisst sind.

14. Prothese gemäss einem der vorhergehenden Ansprüche **dadurch gekennzeichnet, dass** Kugelschalenausschnitt (2) und Krone (5) als Einzelteile ausgestaltet sind und mit einem mechanischen Befestigungsmittel, insbesondere mit einem Schraubgewinde, einem Bajonettverschluss oder einer Klemmvorrichtung, miteinander verbunden sind.

15. Verfahren zur Implantation einer Prothese gemäss einem der vorhergehenden Ansprüche, **dadurch gekennzeichnet, dass** ein Knochen zunächst abgeschält und/oder abgefräst wird, dann für die Krone (5) eine Nut im Kochen geschaffen wird und danach die Prothese (1) auf den Knochen aufgedrückt und/oder aufgeschlagen wird.

16. Materialsatz zur Herstellung einer Prothese gemäss Anspruch 14, **dadurch gekennzeichnet, dass** er verschiedenartige Kugelschalenausschnitte (2) gemäss Anspruch 14 und verschiedenartige Kronen (5) gemäss Anspruch 14 umfasst, wobei die Kugelschalenausschnitte (2) und die Kronen (5) zur Schaffung speziell angepasster Prothesen (1) in verschiedenen Kombinationen jeweils paarweise aneinander befestigbar sind.
